# EUROPEAN PATENT APPLICATION

(11) **EP 4 023 215 A1**
(43) Date of publication of application: **06.07.2022**
(21) Application number: 21217823.0
(22) Date of filing: 27.12.2021
(51) Int. Cl.: A61K 9/20

(54) **A FILM COATED TABLET COMPRISING TERIFLUNOMIDE**

(30) Priority: 30.12.2020 TR 202022336
(71) Applicant: Sanovel Ilac Sanayi ve Ticaret A.S., 34460 Istanbul (TR)
(72) Inventor: DEMIR, Bülent, Istanbul (TR); TOK, Gülcin, Istanbul (TR); SÜNEL, Fatih, Istanbul (TR)
(74) Representative: Sevinç, Erkan

(57) **Abstract**

The present invention relates to a film coated tablet comprising teriflunomide and at least one pharmaceutically acceptable excipient. Further the present invention discloses a method for the preparation of said tablet.

## Description

### Field of the invention

The present invention relates to a film coated tablet comprising teriflunomide and at least one pharmaceutically acceptable excipient. Further the present invention discloses a method for the preparation of said tablet.

### Background of the invention

Multiple Sclerosis (MS) is a demyelinating disease characterized by damage to insulating ends of neurons in the brain and spinal cord. This damage impairs the communication between the components of central nervous system and leads to a series of signs and symptoms including physical, mental, and sometimes, psychiatric problems. Specific symptoms include diplopia, blindness in one eye, muscle weakness, sensory impairment or coordination problems. MS presents with new symptoms either in isolated attacks (in relapsing-remitting forms) or over time (progressively). Symptoms may completely resolve in between attacks. In addition, the likelihood of having permanent neurological damage is high especially as the disease progresses.

Teriflunomide is a pyrimidine synthesis inhibitor. Teriflunomide acts via the mechanism of a dihydroorotate dehydrogenase inhibitor and it is an orally available immunomodulator used in the treatment of relapsing-remitting multiple sclerosis. Chemical name of teriflunomide is (Z)-2-cyano-3-hydroxy-N-[4-(trifluoromethyl)phenyl]but-2enamide and its chemical structure is shown in Formula I.

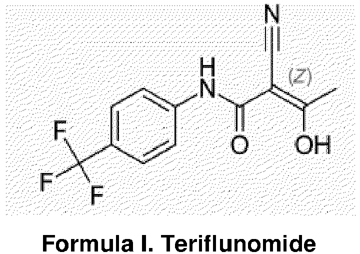

Teriflunomide inhibits rapidly dividing cells including activated T cells that are believed to drive the disease process in MS and it may decrease the risk of infection thanks to its more limited effects on the immune system compared to chemotherapeutic agents.

The document EP0527736B1 is the first molecule patent that describes teriflunomide in the prior art. It mentions the use of teriflunomide in the prophylaxis and/or treatment of rheumatic diseases and the possibility to administer the pharmaceuticals of the invention orally, topically, rectally and parenterally, if required.

US8802735 application discloses a solid pharmaceutical composition of Teriflunomide. The patent teaches that teriflunomide tablets cannot be made with colloidal silicon dioxide as it leads to an unstable product. The patent teaches that teriflunomide tablets without colloidal silicon dioxide display significantly reduced formation of degradants, compared to Teriflunomide tablets containing colloidal silicon dioxide.

In the prior art, compositions comprising teriflunomide show a slightly less degradation of teriflunomide but cannot shed a light on stability and solubility problems. According to the prior art as provided above, there is still a need for stable and highly soluble teriflunomide formulations.

### Detailed description of the Invention

The main object of the present invention is to obtain a film coated tablet comprising teriflunomide that eliminate the above-mentioned problems and provide additional advantages to the relevant prior art.

Another object of the present invention is to obtain a film coated tablet of teriflunomide with high stability and the desired solubility profile.

According to this embodiment of the present invention, a film coated tablet comprising teriflunomide, wherein the amount of teriflunomide is between 2.0% and 15.0% by weight in the total tablet and at least one pharmaceutically acceptable excipient.

According to this embodiment of the present invention, the amount of teriflunomide is between 4.0% and 10.0% by weight in the total tablet

We have found that teriflunomide having the following particle sizes is very important for formulation. Especially, it positively affects the dissolution properties and powder homogenization. The obtained tablets have the desired dissolution profile. The powder is more homogeneous. The content uniformity of the tablets obtained from the more homogeneous powder is more ideal. Therefore, it provides better bioavailability.

As used here in, 'particle size' means the cumulative volume size distrubition as tested by any conventionally accepted method such as the laser diffraction method (i.e. malvern analysis). The term d (0.9) means the size at which %90 by volume of the particles are finer.

According to this embodiment of the present invention, teriflunomide has a d (0.9) particle size less than 20 µm, preferably less than 15 µm, more preferably less than 10 µm.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agents and the excipients.

According to this embodiment of the present invention, at least one pharmaceutically acceptable excipient is selected from the group comprising fillers, binders, disintegrants, lubricants or mixtures thereof.

Suitable fillers are selected from group comprising lactose monohydrate, ammonium alginate, calcium carbonate, calcium phosphate, calcium phosphate dehydrate, neutral pellets, calcium sulfate, cellulose, cellulose acetate, dextrose, erythritol, ethyl cellulose, fructose, glyceryl palmitostearate, lactose, mannitol, magnesium carbonate, magnesium oxide, maltodextrin, maltose, polydextrose, polymethacrylates, polyvinylpyrrolidone, simethicone, sodium alginate, sodium chloride, sorbitol, starch, sucrose, sugar spheres, sulfobutylether beta-cyclodextrin or mixtures thereof.

According to this embodiment of the present invention, the amount of fillers is between 25.0% and 40.0% by weight of the total formulation. Preferably, the amount of fillers is between 29.0% and 37.0% by weight of the total formulation.

According to this embodiment of the present invention, the filler is lactose monohydrate.

Suitable binders are selected from the group comprising corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binders is between 1.0% and 40.0% by weight of the total formulation. Preferably, the amount of binders is between 29.0% and 35.0% by weight of the total formulation.

According to this embodiment of the present invention, the binder is corn starch or hydroxypropyl cellulose or mixtures thereof.

According to this embodiment of the present invention, the amount of corn starch is between 20.0% and 35.0% by weight of the total formulation.

According to this embodiment of the present invention, the amount of hydroxypropyl cellulose is between 1.0% and 5.0% by weight of the total formulation.

According to this embodiment of the present invention, at least two binder is used in the formulation.

Suitable disintegrants are selected from the group comprising sodium starch glycolate, microcrystalline cellulose, croscarmellose sodium, crospovidone, alginates, magnesium aluminum silica, sodium carboxymethyl cellulose, calcium silicate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

According to this embodiment of the present invention, the amount of disintegrants is between 5.0% and 40.0% by weight of the total formulation. Preferably, the amount of disintegrants is between 20.0% and 30.0% by weight of the total formulation.

In the film coated tablet, the ratio of disintegrant to a teriflunomide is in the range of between 1:4 and 4:1, preferably between 1:3 and 3:1. The ratio provide the desired dissolution profile.

According to this embodiment of the invention, the disintegrant is sodium starch glycolate or microcrystalline cellulose or mixtures thereof.

According to this embodiment of the present invention, the amount of sodium starch glycolate is between 5.0% and 15.0% by weight of the total formulation.

According to this embodiment of the present invention, the amount of microcrystalline cellulose is between 5.0% and 25.0% by weight of the total formulation.

According to this embodiment of the invention, at least two disintegrant is used in the formulation.

Suitable lubricants are selected from the group comprising talc, magnesium stearate, calcium stearate, zinc stearate, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium stearyl fumarate, sodium lauryl sulphate or mixtures thereof.

According to this embodiment of the present invention, the amount of lubricants is between 0.1% and 8.0% by weight of the total formulation. Preferably, the amount of lubricants is between 2.0% and 6.0% by weight of the total formulation.

According to this embodiment of the invention, the lubricant is talc or magnesium stearate or mixtures thereof.

Lubricants such as talc improve flow properties of powder by decreasing interparticulate friction, by decreasing van der Waals forces and electrostatic charges, by changing particle size distribution, and by decreasing the effect of humidity on surfaces of host particles by forming a mechanical barrier. We found that talc is a very good lubricant for tablet comprising teriflunomide. Especially, in the present invention, using talc is provided the desired stability. Also, teriflunomide can stick to the punch during manufacturing. This can cause problems during manufacturing. Using lubricant especially talc provides to overcome compressibility and stick problems of active agent.

According to this embodiment of the present invention, the amount of talc is between 1.0% and 6.0% by weight of the total formulation.

According to this embodiment of the present invention, the amount of magnesium stearate is between 0.1% and 2.0% by weight of the total formulation.

According to this embodiment of the invention, at least two lubricant is used in the formulation.

According to this embodiment of the invention, the film coated tablet is free of colloidal silicon dioxide.

Colloidal silicon dioxide acts both as a lubricant or glidant in solid oral pharmaceutical compositions. But, it can cause stability problems in pharmaceutical compositions depending on its amount therein due to its hygroscopic structure that enables absorbing a high amount of water. Although it is frequently used in formulations due to its fluidity, the fact that it increases the rate of becoming unstable by promoting degradation and has highly humectant properties reveals the reasons for not preferring colloidal silicon dioxide especially in compositions that are sensitive to humidity.

In the present invention, the tablet comprises film coating to protect the composition against the moisture and light to maintain the stability. Suitable film coating agents are selected from the group comprising hydroxypropylmethylcellulose, polyethylene glycol (PEG), talc, titanium dioxide, polyvinyl alcohol (PVA), polyvinyl alcohol-polyethylene glycol copolymers, polyvinylprolidone, lecithin, polyvinylprolidone-vinyl acetate copolymer (PVP-VA), pigments, dyes, iron oxide or mixtures thereof.

The film coated tablet of the present invention can be prepared, using standard techniques and manufacturing processes well known in the art, such as direct compression, wet or dry granulation, hot melt granulation, hot melt extrusion, fluidized bed granulation, extrusion/spheronization, slugging, spray drying and solvent evaporation.

It has surprisingly been found that the film coated tablet of excellent uniformity and showing improved compressibility can be obtained when teriflunomide and all excipients (except for lubricants) are formulated together in wet granulation process. Wet granulation process efficiently counteracts segregation, so it can achieve good dissolution and disintegration properties. Solvents are used in the process.

According to this embodiment of the invention, the film coated tablet is prepared with using wet granulation.

Suitable solvents are selected from a group comprising pure water, ethyl alcohol, glycerin, sorbitol, polyethylene glycol, propylene glycol, isopropyl alcohol, or mixtures thereof, preferably granulation solution is pure water.

### Example 1: A film coated tablet comprising teriflunomide

| **Ingredients** | **Amount (% by weight of the total)** |
|---|---|
| Teriflunomide | 2.0 - 15.0 |
| Lactose monohydrate | 25.0 - 40.0 |
| Corn starch | 20.0 - 35.0 |
| Hydroxypropyl cellulose | 1.0 - 5.0 |
| Microcrystalline cellulose | 5.0 - 25.0 |
| Sodium starch glycolate | 5.0 - 15.0 |
| Talc | 1.0 - 6.0 |
| Magnesium stearate | 0.1 -2.0 |
| Film coating agents | 1.0 - 6.0 |
| **Total composition** | **100** |

### Example 2: A film coated tablet comprising teriflunomide

| **Ingredients** | **Amount (% by weight of the total)** |
|---|---|
| Teriflunomide | 9.0 |
| Lactose monohydrate | 30.6 |
| Corn starch | 27.7 |
| Hydroxypropyl cellulose | 2.25 |
| Microcrystalline cellulose | 13.5 |
| Sodium starch glycolate | 9.67 |
| Talc | 3.5 |
| Magnesium stearate | 0.3 |
| Film coating agents | 3.22 |
| **Total composition** | **100** |

### Example 3: A film coated tablet comprising teriflunomide

| **Ingredients** | **Amount (% by weight of the total)** |
|---|---|
| Teriflunomide | 4.5 |
| Lactose monohydrate | 35.1 |
| Corn starch | 27.7 |
| Hydroxypropyl cellulose | 2.25 |
| Microcrystalline cellulose | 13.5 |
| Sodium starch glycolate | 9.67 |
| Talc | 3.5 |
| Magnesium stearate | 0.3 |
| Film coating agents | 3.22 |
| ***Total composition*** | **100** |

### A process for example 1 or 2 or 3;

a) Mixing teriflunomide, lactose monohydrate, corn starch, a half of microcrystalline cellulose, a half of sodium starch glycolate and hydroxypropyl cellulose,
b) Granulating the mixture with pure water,
c) Drying the mixture in fluidized bed at 45°C,
d) Sieving the mixture,
e) Sieving talc, the remaining part of microcrystalline cellulose and the remaining part of sodium starch glycolate through 800 µm and then adding into step (d) and mixing,
f) Adding magnesium stearate and mixing,
g) Compressing the mixture to form tablets,
h) Coating the tablets with film coating comprising film coating agents.

### Film coating agents

| |
|---|
| Hydroxypropyl methylcellulose |
| Titanium dioxide |
| Macrogol/Polyethylene glycol |
| FD&C BLUE #2/INDIGO CARMINE AL |

## Claims

1. A film coated tablet comprising teriflunomide, wherein the amount of teriflunomide is between 2.0% and 15.0% by weight in the total tablet and at least one pharmaceutically acceptable excipient.

2. The film coated tablet according to claim 1, wherein teriflunomide has a d (0.9) particle size less than 20 µm, preferably less than 15 µm, more preferably less than 10 µm.

3. The film coated tablet according to claim 1, wherein at least one pharmaceutically acceptable excipient is selected from the group comprising fillers, binders, disintegrants, lubricants or mixtures thereof.

4. The film coated tablet according to claim 3, wherein fillers are selected from group comprising lactose monohydrate, ammonium alginate, calcium carbonate, calcium phosphate, calcium phosphate dehydrate, neutral pellets, calcium sulfate, cellulose, cellulose acetate, dextrose, erythritol, ethylcellulose, fructose, glyceryl palmitostearate, lactose, mannitol, magnesium carbonate, magnesium oxide, maltodextrin, maltose, polydextrose, polymethacrylates, polyvinylpyrrolidone, simethicone, sodium alginate, sodium chloride, sorbitol, starch, sucrose, sugar spheres, sulfobutylether beta-cyclodextrin or mixtures thereof.

5. The film coated tablet according to claim 3, wherein binders are selected from the group comprising corn starch, hydroxypropyl cellulose, gelatin, alginates, carbomers, carboxymethylcellulose sodium, starch, pregelatinized starch, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

6. The film coated tablet according to claim 5, wherein the binder is corn starch or hydroxypropyl cellulose or mixtures thereof.

7. The film coated tablet according to claim 3, wherein disintegrants are selected from the group comprising sodium starch glycolate, microcrystalline cellulose, croscarmellose sodium, crospovidone, alginates, magnesium aluminum silica, sodium carboxymethyl cellulose, calcium silicate, carboxymethyl cellulose, calcium carboxymethyl cellulose, low substituted hydroxypropyl cellulose, polyacryline potassium, poloxamer, sodium alginate, sodium glycine carbonate, sodium dodecyl sulfate or mixtures thereof.

8. The film coated tablet according to claim 7, wherein the ratio of disintegrant to a teriflunomide is in the range of between 1:4 and 4:1, preferably between 1:3 and 3:1.

9. The film coated tablet according to claim 7, wherein the disintegrant is sodium starch glycolate or microcrystalline cellulose or mixtures thereof.

10. The film coated tablet according to claim 3, wherein lubricants are selected from the group comprising talc, magnesium stearate, calcium stearate, zinc stearate, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium stearyl fumarate, sodium lauryl sulphate or mixtures thereof.

11. The film coated tablet according to claim 10, wherein the lubricant is talc or magnesium stearate or mixtures thereof.

12. The film coated tablet according to claim 10, wherein the amount of talc is between 1.0% and 6.0% by weight of the total formulation.

13. The film coated tablet according to claim 10, wherein at least two lubricant is used in the formulation.

14. The film coated tablet according to claim 1, wherein the film coated tablet is free of colloidal silicon dioxide.

15. The film coated tablet according to claim 1, wherein the tablet is prepared with using wet granulation.
